# EUROPEAN PATENT APPLICATION

(11) **EP 4 717 256 A1**
(43) Date of publication of application: **01.04.2026**
(21) Application number: 25201623.3
(22) Date of filing: 11.09.2025
(51) Int. Cl.: A61K 9/00, A61K 9/20, A61K 31/00, A61K 9/14, A61K 9/28, A61K 9/50

(54) **PHARMACEUTICAL COMPOSITION COMPRISING ROSUVASTATIN AND OMEGA-3, PROCESS FOR PREPARING THE SAME, AND USES THEREOF**

(30) Priority: 12.09.2024 MX 2024011226
(71) Applicant: Laboratorios Silanes, S.A. de C.V., Mexico, 11000 (MX)
(72) Inventor: OLLERVIDES RUBIO, Paola Yazmín, 11000 Ciudad de México (MX); GONZÁLEZ CANUDAS, Jorge Alejandro, 11000 Ciudad de México (MX); RIOS BRITO, Kevin Francisco, 11000 Ciudad de México (MX); CUAHUTENCOS ESCOBAR, Ernesto, 11000 Ciudad de México (MX)
(74) Representative: Hoffmann Eitle

(57) **Abstract**

The present invention provides a pharmaceutical composition comprising a) a first phase comprising rosuvastatin, which is preferably in the form of a coated tablet, and b) a second phase comprising omega-3, which is preferably in the form of granules. The pharmaceutical composition of the present invention solves a set of significant technological challenges due to the physicochemical properties and difference in dosage of each drug. Said pharmaceutical composition is stable, making it safe and effective in the treatment and prevention of disorders associated with lipid metabolism. The invention is also directed to a process for manufacturing the pharmaceutical composition.

## Description

### TECHNICAL FIELD

The present invention relates to the field of the pharmaceutical industry and more particularly to a pharmaceutical composition comprising rosuvastatin and omega-3, a process for preparing the same, and uses thereof.

### BACKGROUND

Dyslipidemia is a medical condition characterized by the presence of abnormal levels of lipids in the blood, specifically cholesterol and triglycerides. This disorder can manifest itself in various ways, such as elevated total cholesterol, increased low-density lipoprotein (LDL) cholesterol, decreased highdensity lipoprotein (HDL) cholesterol, and/or increased triglycerides. Dyslipidemia is a significant risk factor for the development of cardiovascular diseases, which is a serious public health concern.

The causes of dyslipidemia can be primary, secondary, or a combination of both. Primary dyslipidemias are generally genetic in origin and are inherited from one generation to the next. Examples of primary dyslipidemias include familial hypercholesterolemia and familial combined dyslipidemia. On the other hand, secondary dyslipidemias are caused by other medical conditions or lifestyle factors. Among these conditions are diabetes mellitus, hypothyroidism, obesity, metabolic syndrome, and excessive alcohol consumption. Likewise, certain medicaments, such as corticosteroids and oral contraceptives, can also contribute to the onset of dyslipidemia.

Dyslipidemia poses a significant challenge for healthcare systems worldwide due to its strong association with cardiovascular diseases, which are one of the leading causes of morbidity and mortality. Cardiovascular diseases related to dyslipidemia, such as atherosclerosis, coronary heart disease, and stroke, generate a considerable economic burden, both in terms of direct healthcare costs and lost productivity. Also, the diagnosis and management of dyslipidemia require significant resources, including laboratory tests to monitor lipid levels, regular medical consultations, and, in many cases, pharmacological treatments.

The treatment of dyslipidemia focuses on reducing cardiovascular risk by controlling levels of lipids in the blood. Treatment strategies include lifestyle modifications and pharmacological therapies.

Among pharmacological therapies are the administration of statins, fibrates, cholesterol absorption inhibitors, and omega-3 fatty acids. In this regard, statins act by inhibiting the enzyme 3-hydroxy-3-methylglutaryl-CoA reductase (HMG-CoA reductase), which is involved in lipid metabolism, so that these types of drugs are used to reduce cholesterol and triglyceride-rich lipoproteins. However, statins are known to not be effective enough in reducing triglycerides, so treatments involving the co-administration of statins with omega-3 fatty acids have been suggested since these types of fatty acids reduce the availability of free fatty acids needed for triglyceride production.

Specifically, combination therapy with omega-3 and rosuvastatin (statin) has been found to improve endothelial function in patients with dyslipidemia and endothelial dysfunction; it also lowers the lipid profile (triglycerides and low-density lipoproteins).

Accordingly, there is a need for medicaments that provide both active ingredients in a single dosage, improving patient compliance with treatment by simplifying the therapeutic regimen and also reducing the risk of omissions and adverse events.

However, combining a statin and omega-3 poses a technological challenge due to the difference in physicochemical, rheological, and solubility properties of the active ingredients. Specifically, rosuvastatin is a water-soluble drug with poor flow, adhesive and hydrolytic properties, whereas omega-3 is a liquid fat-soluble drug, so the combination could cause interference in stability, absorption, mechanism of release, and bioavailability.

In this regard, International Publication No. WO2016/024844 discloses a combined preparation comprising an omega-3 fatty acid ester and a statin (which may be rosuvastatin), which has high stability. According to said document, the stability problem is solved by embedding a tablet comprising a statin in a capsule containing an omega-3 fatty acid ester. Said document discloses that the coated tablet comprising the statin must first be put into the capsule, and then the liquid omega-3 must be injected; the incorporation of omega-3 in liquid form provides a barrier against moisture.

Similarly, International Publication No. WO 2011/150505 discloses a multiphase soft gelatin capsule comprising a solid dosage form comprising a statin with at least one coating; and at least one liquid fill phase comprising omega-3 fatty acids, wherein there is no chemical reaction between the multiple phases. Said document further states that the lipophilic filling of the capsule, which may be omega-3, allows the solid pharmaceutical form to be transported, is also compatible with the soft gelatin coating, and does not interfere with or degrade the solid pharmaceutical form.

Moreover, International Publication No. WO 2012/128587 discloses an oral complex composition comprising a) a soft capsule core containing omega-3; b) a first coating layer which encases the soft capsule core and comprises a hydrophobic coating material; and c) a second coating layer deposited on the first coating layer, which contains i) rosuvastatin or a pharmaceutically acceptable salt thereof, and ii) polyvinyl alcohol, polyvinyl alcohol-polyethylene glycol graft copolymer, or a mixture thereof.

None of the documents described above propose a pharmaceutical composition comprising omega-3 and rosuvastatin that is stable, easy to administer in a single dose, and wherein the omega-3 is included in the form of granules as claimed in the present invention.

As a result of the above, efforts have been made to eliminate the drawbacks related to stability of pharmaceutical compositions comprising rosuvastatin and omega-3 as active ingredients by developing a pharmaceutical composition comprising rosuvastatin and omega-3, a process for preparing the same, and uses thereof, wherein a single, solid, stable, immediate-release pharmaceutical form is provided. The proposed composition and manufacturing process thereof overcome the major drawbacks associated with the combination of active ingredients through its novel manufacturing process.

Thus, the present invention proposes a combination of the active ingredients rosuvastatin and omega-3 into a single dosage unit, thereby solving a set of significant technological challenges due to the physicochemical properties and difference in dose to ensure obtaining a stable product for treating and preventing disorders associated with lipid metabolism.

### OBJECTS OF THE INVENTION

Considering the shortcomings of the prior art, it is an object of the present invention to provide a solid pharmaceutical composition comprising rosuvastatin and omega-3, wherein said pharmaceutical composition is stable, immediate release, and easy to administer.

A further object of the invention is to provide a process for manufacturing a pharmaceutical composition comprising rosuvastatin and omega-3, improving the stability of the active ingredients.

These and other objects are achieved by a pharmaceutical composition comprising rosuvastatin and omega-3, a process for preparing the same, and uses thereof in accordance with the present invention.

### SUMMARY OF THE INVENTION

To this end, a first aspect of the present invention relates to a pharmaceutical composition comprising:
a) a first phase comprising rosuvastatin; and
b) a second phase comprising omega-3,
wherein the first phase comprising rosuvastatin is in the form of a coated tablet, and the second phase comprising omega-3 is in the form of granules.

A second aspect of the invention provides a process for manufacturing the pharmaceutical composition described above, wherein the process comprises the stages of:
a) providing a first phase comprising rosuvastatin, wherein the first phase comprising rosuvastatin is in the form of a coated tablet;
b) providing a second phase comprising omega-3, wherein the second phase comprising omega-3 is in the form of granules; and
c) encapsulating the first phase comprising rosuvastatin and the second phase comprising omega-3.

A third aspect of the invention relates to the use of a pharmaceutical composition of the present invention in treating and preventing disorders associated with lipid metabolism.

### BRIEF DESCRIPTION OF THE FIGURES

The novel aspects considered as characteristic of the present invention will be set forth particularly in the appended claims. However, some embodiments, features, and some objects and advantages thereof will be better understood from the detailed description when read in conjunction with the accompanying drawings, in which:
**Figure 1** is a plot showing the results of the mean plasma concentration versus sampling time.
**Figure 2** depicts a plot showing the results of the mean of log-transformed rosuvastatin concentrations.

### DETAILED DESCRIPTION OF THE INVENTION

It should be understood that the present invention is not limited to the methodologies, protocols, and reagents provided by the detailed description, as these may vary. It should also be understood that the terminology used herein is merely for describing particular embodiments and is not intended to limit the scope of the present disclosure, which will only be limited by the accompanying claims. Unless otherwise defined, all technical and scientific terms used herein have the same meaning as those commonly understood by a person skilled in the art.

As used in the present invention, the term "approximately" refers to a measurable value that, due to the nature of the techniques used to measure it, may vary. In the case of an amount of weight, time, dose, etc., it is understood to encompass, for example, deviations of ± 20% or ± 10%, as another example ± 5%, as another example ± 1%, and as another example + 0.1% from a specified amount, as such deviations are appropriate for carrying out the disclosed process.

The term "pharmaceutically acceptable salt" of a given compound refers to salts that retain the biological efficacy and properties of the given compound and are not biologically or otherwise undesirable. Pharmaceutically acceptable base addition salts can be prepared from inorganic or organic bases. Salts derived from inorganic bases include, by way of example only, sodium, potassium, lithium, ammonium, calcium, and magnesium salts.

As used herein, the term "stability" is defined as the ability of a pharmaceutical product to retain its chemical, physical, microbiological, and biopharmaceutical properties within specified limits throughout its shelf-life.

The present invention relates to solid, stable, immediate-release pharmaceutical compositions that retain dissolution and bioavailability of a statin and omega-3 fatty acids. Specifically, in the compositions of the present invention, said statin is rosuvastatin. In this regard, it is known that the combination of rosuvastatin and omega-3 represents a set of significant technological challenges because the drugs have different rheological and solubility characteristics. In particular, omega-3 is a liquid fat-soluble drug, while rosuvastatin is a water-soluble drug with poor flow, adhesive and hydrolytic properties, so the combination of drugs could cause interference in stability, adsorption, and mechanism of release.

In order to solve the aforementioned technical problem, the present invention provides a pharmaceutical composition comprising:
a) a first phase comprising rosuvastatin; and
b) a second phase comprising omega-3.

As to the first phase comprising rosuvastatin, in a preferred embodiment, it is in the form of a tablet; in a more preferred embodiment, the rosuvastatin tablet is coated.

On the other hand, in a preferred embodiment, the second phase comprising omega-3 is in the form of granules.

Pharmaceutically acceptable excipients are required for the preparation of the pharmaceutical composition, which are selected from the group consisting of diluents, stabilizing agents, disintegrants, glidants, lubricants, solvents, coatings, absorbents, antioxidants, and surfactants.

Examples of pharmaceutically acceptable diluents include, but are not limited to, lactose monohydrate, cellulose derivatives such as microcrystalline cellulose, silicified microcrystalline cellulose; starch derivatives such as pregelatinized starch and corn starch; phosphates such as dibasic calcium phosphate; mannitol, maltitol, dextrose, calcium phosphates, or combinations thereof.

In a non-limiting embodiment, stabilizing agents are selected from the group consisting of phosphates such as dibasic calcium phosphate, citrates, antioxidants such as butylhydroxytoluene, or combinations thereof.

Pharmaceutically acceptable disintegrants include, but are not limited to, cellulose derivatives, for example, croscarmellose sodium, hydroxypropyl celluloses, carboxymethyl celluloses, microcrystalline cellulose; povidone derivatives such as crospovidone, plasdones; starch derivatives such as pregelatinized starch, sodium starch glycolate, and corn starch; or combinations thereof.

Examples of pharmaceutically acceptable glidants include cellulose derivatives such as carboxymethyl celluloses, microcrystalline cellulose; starch derivatives such as pregelatinized starch and corn starch; silicate derivatives such as magnesium silicate, magnesium trisilicate; colloidal silicon dioxide; magnesium stearate; talc; calcium stearate, polyethylene glycol; or combinations thereof.

Examples of pharmaceutically acceptable lubricants and surfactants include, but are not limited to, stearate derivatives such as magnesium stearate, zinc stearate, calcium stearate, stearic acid, monostearates, stearyl fumarate; sulfated derivatives such as sodium lauryl sulfate and magnesium lauryl sulfate; talc; poloxamers; polyoxyethylene castor oil derivatives; benzalkonium chloride; benzetonium chloride; polyoxyethylene ethers; polyoxyethylene sorbitan fatty acid esters; or combinations thereof.

Pharmaceutically acceptable adsorbents include, but are not limited to, aluminum magnesium metasilicate; phosphate derivatives, for example, anhydrous dibasic calcium phosphate; cellulose derivatives such as microcrystalline cellulose; or combinations thereof.

Examples of antioxidants include, but are not limited to, propyl gallate, tocopherols, hydroxytoluene (BHT), butylhydroxyanisole (BHA), tert-butylhydroquinone (TBHQ), alpha-tocopherols, and combinations thereof.

Said first phase comprising rosuvastatin further comprises at least one of a diluent, a stabilizing agent, a disintegrant, a glidant, and a lubricant.

In a preferred embodiment, the first phase comprising rosuvastatin further comprises at least one of a diluent selected from the group consisting of lactose monohydrate, microcrystalline cellulose, anhydrous calcium phosphate, and corn starches; a stabilizing agent selected from the group consisting of phosphate derivatives including anhydrous dibasic calcium phosphate, and aluminum magnesium silicate; a disintegrant selected from the group consisting of croscarmellose sodium, cellulose derivatives including hydroxypropyl celluloses, carboxymethyl celluloses, microcrystalline cellulose, povidone derivatives including plasdones, starch derivatives including pregelatinized starch, sodium starch glycolate, and corn starch; a glidant selected from the group consisting of colloidal silicon dioxide, cellulose derivatives including carboxymethyl celluloses, microcrystalline cellulose, starch derivatives including pregelatinized starch and corn starch, silicate derivatives including magnesium silicate, magnesium trisilicate, and talc; a lubricant selected from the group consisting of stearate derivatives including zinc stearate, magnesium stearate, calcium stearate, stearic acid, monostearates, stearyl fumarate, sulfated derivatives including magnesium lauryl sulfate, and talc; and a solvent which is water.

In a more preferred embodiment, the first phase comprising rosuvastatin further comprises two lubricants, wherein the first lubricant is talc, and the second lubricant is zinc stearate.

In an even more preferred embodiment, the first phase comprising rosuvastatin further comprises:
- rosuvastatin or a pharmaceutically acceptable salt thereof as an active ingredient in a dose of from 10 mg to 20 mg;
- lactose monohydrate in an amount of from 40% to 80% by weight of the tablet;
- dibasic calcium phosphate in an amount of from 0.26% to 2% by weight of the tablet;
- croscarmellose sodium in an amount of from 0.5% to 5% by weight of the tablet;
- colloidal silicon dioxide in an amount of from 0.5% to 6% by weight of the tablet;
- talc in an amount of from 0.2% to 5% by weight of the tablet; and
- zinc stearate in an amount of from 0.5% to 5% by weight of the tablet.

The rosuvastatin in the first phase is preferably in its calcium form, wherein 10.4 mg rosuvastatin calcium is equivalent to a dose of 10 mg rosuvastatin.

In another preferred embodiment, the rosuvastatin in the first phase is preferably in its calcium form, wherein 20.80 mg rosuvastatin is equivalent to 20 mg rosuvastatin.

In a preferred embodiment, the amount of the active ingredient rosuvastatin is adjusted according to assessment.

As to the coating, it acts as a protective barrier between the active ingredients and as a barrier against moisture, retaining the stability of a composition. In a non-limiting embodiment, the coating is selected from the group consisting of cellulose derivatives such as hydroxypropyl methylcellulose, hydroxypropyl cellulose, carboxymethyl celluloses; polyvinyl derivatives such as polyvinyl acetate; polyethylene glycol, povidones in all K grades and derivatives thereof. In one embodiment, the coating is polyvinyl acetate; in a preferred embodiment, the coating is Opadry, such as Opadry AMB II white 88A180021. The coating is present in a pharmaceutically acceptable amount in the range of 0.5% to 10% by weight of the rosuvastatin tablet; in a preferred embodiment, the coating is present in an amount in the range of 0.5% to 6% by weight of the rosuvastatin tablet. To incorporate the coating, as will be described below, water is used as a solvent; however, it evaporates during the process.

On the other hand, in a preferred embodiment, the second phase comprising omega-3 is in the form of granules that further comprise at least one of an adsorbent, a diluent, an antioxidant, a disintegrant, and a surfactant.

In another preferred embodiment, the second phase comprising omega-3 further comprises at least one of an adsorbent selected from the group consisting of phosphate derivatives including dibasic calcium phosphate, aluminum magnesium silicate, aluminum magnesium metasilicate, and cellulose derivatives including microcrystalline cellulose; a diluent selected from the group consisting of cellulose derivatives including microcrystalline cellulose, silicified microcrystalline cellulose, phosphate derivatives including dibasic calcium phosphate, and starch derivatives including pregelatinized starch and corn starch; an antioxidant selected from the group consisting of propyl gallate, tert-butylhydroquinone (TBHQ), butylhydroxytoluene (BHT), butylhydroxyanisole (BHA), and alpha-tocopherols; a disintegrant selected from the group consisting of cellulose derivatives including hydroxypropyl celluloses, carboxymethyl celluloses, microcrystalline cellulose, povidone derivatives including crospovidone and plasdone, and starch derivatives including pregelatinized starch, sodium starch glycolate, and corn starch; and a surfactant selected from the group consisting of sodium lauryl sulfate, poloxamers, polyoxyethylene castor oil derivatives, benzalkonium chloride, benzetonium chloride, polyoxyethylene alkyl ethers, and polyoxyethylene sorbitan fatty acid esters.

In a more preferred embodiment, the adsorbent is aluminum magnesium metasilicate, the diluent is silicified microcrystalline cellulose, the antioxidant is propyl gallate, the disintegrant is crospovidone, and the surfactant is sodium lauryl sulfate.

In an even more preferred embodiment, the second phase comprising omega-3 and which is in the form of granules further comprises:
- omega 3 in an amount of 300 mg ± 0.5 mg;
- aluminum magnesium metasilicate in an amount of from 20% to 50% by weight of the granules;
- silicified microcrystalline cellulose in an amount of from 1% to 5% by weight of the granules;
- propyl gallate in an amount of from 0.5% to 5% by weight of the granules;
- crospovidone in an amount of from 3% to 10% by weight of the granules; and
- sodium lauryl sulfate in an amount of from 0.5% to 5% by weight of the granules.

In a preferred embodiment, 300 mg of omega-3 is equivalent to 220 mg of fatty acid esters; in a more preferred embodiment, 300 mg of omega-3 is equivalent to 125 mg of eicosapentaenoic acid (EPA) ethyl ester and to 95 mg of docosahexaenoic acid (DHA) ethyl ester.

In one embodiment, the pharmaceutical composition of the present invention is a solid form, preferably a capsule.

The pharmaceutical composition as described above is notable for its physical, chemical, and biological stability, as well as for its controlled drug release and low cost.

Surprisingly, the pharmaceutical composition of the present application was found to be highly stable, since, in a 6-month accelerated stability study, the pharmaceutical composition of the present invention retained its chemical and physical characteristics within the established specifications, maintaining its quality, efficacy, and safety. This is also due to the fact that the pharmaceutical composition does not affect the release mechanism of rosuvastatin in the presence of omega-3, ensuring the therapeutic effect of both drugs.

The pharmaceutical composition of the present invention is useful in the prevention and treatment of disorders associated with lipid metabolism. In this regard, combination therapy with omega-3 and rosuvastatin (statin) was found to improve endothelial function in patients with dyslipidemia and endothelial dysfunction; it also reduces the lipid profile (triglycerides and low-density lipoproteins). Likewise, in a clinical trial sponsored by Kuhnil Pharmaceutical Co Ltd, it was found that a treatment comprising the administration of rosuvastatin and omega-3 in patients with residual hypertriglyceridemia resulted in a greater reduction in the concentration of triglycerides and low-density lipoproteins in the blood, compared to the administration of a statin alone (rosuvastatin).

Accordingly, the present invention further provides a method for preventing and treating disorders associated with lipid metabolism, which comprises administering a pharmaceutical composition according to the present invention, wherein the lipid metabolism disorder is selected from the group consisting of dyslipidemia, atherosclerosis, coronary heart disease, cardiovascular disease, and pancreatitis. Within dyslipidemias, familial hypercholesterolemia, hypertriglyceridemia, familial combined dyslipidemia, familial chylomicronemia syndrome, and hypoalphalipoproteinemia can be found.

In a preferred embodiment, the treatment method comprises administering a composition according to the present invention once a day.

The present invention also discloses an optimized process that allows the manufacture of a pharmaceutical composition as described above.

The process for manufacturing the pharmaceutical composition as described above comprises the following stages:
a) providing a first phase comprising rosuvastatin, wherein the first phase comprising rosuvastatin is in the form of a coated tablet;
b) providing a second phase comprising omega-3, wherein the second phase comprising omega-3 is in the form of granules; and
c) encapsulating the first phase comprising rosuvastatin and the second phase comprising omega-3.

The manufacture of the first phase comprising rosuvastatin comprises the following steps:
- mixing the rosuvastatin with a glidant, a disintegrant, a stabilizing agent, and a diluent;
- lubricating the above mixture with at least one lubricant;
- compressing the lubricated mixture to obtain a rosuvastatin tablet;
- coating the tablet.

In a non-limiting embodiment, the diluent is selected from the group consisting of lactose monohydrate, microcrystalline cellulose, anhydrous calcium phosphate, and corn starches; the stabilizing agent is selected from the group consisting of phosphate derivatives including anhydrous dibasic calcium phosphate, and aluminum magnesium silicate; the disintegrant is selected from the group consisting of croscarmellose sodium, cellulose derivatives including hydroxypropyl celluloses, carboxymethyl celluloses, microcrystalline cellulose, povidone derivatives including plasdones, starch derivatives including pregelatinized starch, sodium starch glycolate, and corn starch; the glidant is selected from the group consisting of colloidal silicon dioxide, cellulose derivatives including carboxymethyl celluloses, microcrystalline cellulose, starch derivatives including pregelatinized starch and corn starch, silicate derivatives including magnesium silicate, magnesium trisilicate, and talc; the lubricant is selected from the group consisting of stearate derivatives including zinc stearate, magnesium stearate, calcium stearate, stearic acid, monostearates, stearyl fumarate, sulfated derivatives including magnesium lauryl sulfate, and talc; the solvent is water; and the coating is selected from the group consisting of cellulose derivatives including hydroxypropyl methylcellulose, hydroxypropyl cellulose, carboxymethyl celluloses, polyvinyl derivatives including polyvinyl alcohol, polyethylene glycol, povidones in all K grades and derivatives thereof, and polyvinyl acetate coatings.

Additionally, to incorporate the coating, it is dispersed in water, which evaporates during the process.

As indicated above, the second phase comprising omega-3 corresponds to granules of omega-3 granules, which do not have an enteric coating.

To prepare the second phase comprising omega-3, the following steps are carried out:
- mixing the omega-3 with an adsorbent, an antioxidant, a surfactant, a diluent, and a disintegrant; and
- granulating.

In a non-limiting embodiment, the adsorbent is selected from the group consisting of phosphate derivatives including dibasic calcium phosphate, aluminum magnesium silicate, aluminum magnesium metasilicate, and cellulose derivatives including microcrystalline cellulose; the diluent is selected from the group consisting of cellulose derivatives including microcrystalline cellulose, silicified microcrystalline cellulose, phosphate derivatives including dibasic calcium phosphate, and starch derivatives including pregelatinized starch and corn starch; the antioxidant is selected from the group consisting of propyl gallate, tert-butylhydroquinone (TBHQ), butylhydroxytoluene (BHT), butylhydroxyanisole (BHA), and alpha-tocopherols; the disintegrant is selected from the group consisting of cellulose derivatives including hydroxypropyl celluloses, carboxymethyl celluloses, microcrystalline cellulose, povidone derivatives including crospovidone and plasdone, and starch derivatives including pregelatinized starch, sodium starch glycolate, and corn starch; and the surfactant is selected from the group consisting of sodium lauryl sulfate, poloxamers, polyoxyethylene castor oil derivatives, benzalkonium chloride, benzetonium chloride, polyoxyethylene alkyl ethers, and polyoxyethylene sorbitan fatty acid esters.

On the other hand, the encapsulation stage comprises putting the first phase comprising rosuvastatin and the second phase comprising omega-3 into a gelatin capsule.

Due to the novel process and formulation of the medicament, it was possible to obtain a product with demonstrably improved stability through studies conducted according to current regulatory guidelines, in which quality attributes were met during the assessment period. Stability testing is a means of comparing different formulations, packaging materials, or manufacturing processes in short-term experiments. Once the final formulation and manufacturing process have been established, the manufacturer conducts a series of stability tests to predict the product or drug stability in this case and determine shelf-life and storage conditions thereof.

The initial results and results under accelerated conditions for determining the stability of compositions of the present invention for the manufacture of a medicament useful in the prevention and treatment of disorders associated with lipid metabolism.

Technological development consisted of a number of assessments defining the formulation and process as described below. The skilled person in the art will realize that multiple variations and embodiments are possible in performing the present invention without departing from the spirit and scope thereof to ensure proper product function and to meet the required quality characteristics.

The present invention will be better understood from the following examples, which are presented solely for illustrative purposes to allow a thorough understanding of the preferred embodiments of the present invention, without implying that there are no other embodiments not illustrated that can be put into practice based on the detailed description.

### EXAMPLES

### Example 1. Process for manufacturing a pharmaceutical composition according to one embodiment of the invention

A pharmaceutical composition comprising rosuvastatin and omega-3 at a concentration of 10 mg/300 mg was manufactured, the qualitative and quantitative formula of which is detailed in Table 1.

**Table 1. Qualitative and quantitative formulas of the pharmaceutical composition**

| **Component** | **Amount** | | **Function** |
|---|---|---|---|
| **Phase I** | | | |
| Rosuvastatin calcium | 10.40 mg | Equivalent to 10.00 mg of rosuvastatin | Active ingredient |
| Lactose monohydrate | 50.20 mg | DCL 11 for adjustment of total solids to 73.00 mg/tablet | Diluent |
| Dibasic calcium phosphate | 1.20 mg | Anhydrous | Stabilizing agent |
| Croscarmellose sodium | 3.50 mg | | Disintegrant |
| Colloidal silicon dioxide | 0.40 mg | | Lubricant |
| Talc | 3.60 mg | | Lubricant |
| Zinc stearate | 0.70 mg | | Lubricant |
| Purified water | q.s. | It evaporates during the process | Solvent |

| Coating | | | |
|---|---|---|---|
| Opadry | 3.00 mg | Opadry AMB II White 88A180021 | Coating system |
| | | Composition: | |
| | | Partially hydrolyzed polyvinyl alcohol 39.50% | |
| | | Talc ............................................................. 38.00% | |
| | | Titanium dioxide..........................................15.00% | |
| | | GMCC type I ................................................. 4.50% | |

| **Component** | **Amount** | | **Function** |
|---|---|---|---|
| | | Sodium lauryl sulfate.......................................3.00% | |

| **Phase II** | | | |
|---|---|---|---|
| Omega-3 | 300.00 mg | It is added as granules of omega-3 | Active ingredient |
| equivalent to | 220 mg | | |
| eicosapentaenoic acid (EPA) ethyl ester | 125 mg | | |
| docosahexaenoic acid (DHA) ethyl ester | 95 mg | | |
| Aluminum magnesium metasilicate | 171.50 mg | | Adsorbent |
| Silicified microcrystalline cellulose | 21.00 mg | SMCC 90 | Diluent |
| Propyl gallate | 15.00 mg | | Antioxidant |
| Crospovidone | 49.50 mg | | Disintegrant |
| Sodium lauryl sulfate | 15.00 mg | | Surfactant |

| **Capsule** | | | |
|---|---|---|---|
| Capsule | 124.00 mg | #00 Capsule with opaque white body and opaque white cap | Container for pharmaceutica l form |

To manufacture the pharmaceutical compositions according to one embodiment of the present invention as described above, a process generally consisting of three stages described below was carried out:

### I. Preparation of phase 1 comprising rosuvastatin:

The aim of this stage is to prepare a first phase comprising rosuvastatin, for which the following steps were carried out:
1. Rosuvastatin calcium (active ingredient) was mixed with a glidant (colloidal silicon dioxide), a disintegrant (croscarmellose sodium), and a stabilizing agent (anhydrous dibasic calcium phosphate) for approximately 8 minutes. Excipients were previously sieved.
2. A sieving operation was performed on the mixture using a 0.045-inch (1.143 mm) mesh.
3. The sifted material from step No. 2 was mixed with a diluent (lactose monohydrate DCL 11) for approximately 5 minutes.
4. The mixture from step No. 3 was mixed with a first lubricant (talc) previously sieved for approximately 3 minutes.
5. The mixture from step No. 4 was mixed with a second lubricant (zinc stearate) for approximately 3 minutes.
6. The lubricated mixture obtained in step No. 5 was compressed into 70 ± 7.0 mg cores, with a hardness of 3.0-8.0 kp and a disintegration time of less than 5 minutes.
7. The cores obtained in step No. 6 were coated with a polyvinyl acetate coating, which is Opardy AMB II white 88A180021.

The result of the above steps is a white colored, biconvex, coated tablet plain on both sides with an average weight: 73.0 mg ± 7.0 mg; a thickness: 3.0 mm to 3.6 mm; and a disintegration time of no more than 5 min in water at 37 °C ± 2 °C.

### II. Preparation of phase 2 comprising omega-3:

The aim of this stage is to prepare a second phase comprising omega-3, for which the following steps were carried out:
1. Omega-3 (active ingredient), an adsorbent (aluminum and magnesium metasilicate), and an antioxidant (propyl gallate) were mixed for approximately 13 minutes in a granulation equipment.
2. Approximately 50% of a previously sifted surfactant (sodium lauryl sulfate) was added and mixed for approximately 7 min.
3. A previously sifted diluent (silicified microcrystalline cellulose) and a previously sifted disintegrant (crospovidone) were added and mixed for approximately 7 minutes.
4. The remainder (approximately 50%) of the surfactant (sodium lauryl sulfate) was added.

### III. Encapsulation

To encapsulate, and therefore, prepare a pharmaceutical composition according to the present invention, a size #00 capsule with opaque white body and opaque white cap was used. To this end, said phase I comprising rosuvastatin and said phase II comprising omega-3 were put into said gelatin capsule.

Average weight of capsule (capsule + granules) was 969.0 mg ± 57.0 mg; average weight of contents (granules + tablet) was 645.0 mg ± 57.0 mg; average weight of filled capsule was 769.0 mg ± 57.0 mg; disintegration time was less than 15 min in water at 37 °C ± 2.0 °C.

Finally, the pharmaceutical composition was packaged in a primary container consisting of a cold-formed aluminum foil blister pack 176 µm - aluminum foil 25 µm, which provides airtightness; the secondary container is a white cardboard box.

### Example 2. Stability of a pharmaceutical composition according to a preferred embodiment of the present invention.

The aim of this study was to assess the stability of a pharmaceutical composition according to a preferred embodiment of the present invention, particularly a composition as described in Example 1, Table 1 under accelerated stability conditions (40 °C ± 2 °C / 75% ± 2% relative humidity) for 6 months.

The study began on March 03, 2023, and ended on September 03, 2023, in compliance with Section 11 of Mexican Official Standard NOM-059-SSA1-2015, Good Manufacturing Practices for Medicines, and in accordance with Section 7.1 of NOM-073-SSA1-2015, Stability of Drugs and Medicines, as well as Herbal Remedies.

During the study, an assessment was performed on three batches (DFF2208-22, DFF2208-23, and DFF2208-24) of the pharmaceutical composition described in Table 1 and packaged in a primary container consisting of a cold-formed aluminum foil blister pack 176 µm - aluminum foil 25 µm; the secondary container being a white cardboard box.

Initial sampling was performed at the end of the first month (March 03, 2023), third month (June 03, 2023), and six months (September 03, 2022). The following samples were considered for each analysis period:
- 40 blister packs for physicochemical testing.
- 32 blister packs for microbiological testing for the initial and final periods.

The parameters analyzed were product description, rosuvastatin content, omega-3 content, rosuvastatin dissolution, organic impurities in rosuvastatin, microbial limits (initial and final periods), and water content (tablet and granules).

Tables 2, 3, and 4 show the results obtained for the three batches assessed.

**Table 2. Results of stability study for batch DFF2208-22**

| **Determination** | **Specification** | **Sample No.** | **Initial Mar 3, 2023** | **1 month Apr 3, 2023** | **3 months June 3, 2023** | **6 months September 3, 2023** |
|---|---|---|---|---|---|---|
| Rosuvastatin Content | Rosuvastatin 90.0% to 110.0% 9.0 to 11.0 mg/capsule | M1 | 96.26% | 99.05% | 102.52% | 92.37% |
| | | M2 | 95.10% | 99.58% | 101.27% | 92.54% |
| | | M3 | 96.22% | 99.13% | 101.89% | 92.04% |
| | | M_{avg} | 95.9% | 99.3% | 101.9% | 92.3% |
| | | CV | 0.69% | 0.29% | 0.62% | 0.28% |
| | | mg | 9.6 mg/capsule | 9.9 mg/capsule | 10.2 mg/capsule | 9.2 mg/capsule |
| Omega-3 Content | EPA 90% to 110.0% 112.5 mg to 137.5 mg per capsule | M1 | 108.99% | 109.27% | 103.08% | 97.74% |
| | | M2 | 109.10% | 108.96% | 102.98% | 97.74% |
| | | M3 | 109.78% | 109.62% | 103.21% | 100.70% |
| | | M_{avg} | 109.3% | 109.3% | 103.1% | 98.7% |
| | | CV | 0.40 | 0.30 | 0.11 | 1.74% |
| | | mg | 136.6 mg/capsule | 136.6 mg/capsule | 128.9 mg/capsule | 123.4 mg/capsule |
| | DHA 90.0% to 110.0% | M1 | 109.10% | 109.34% | 102.83% | 97.99% |
| | | M2 | 108.97% | 108.58% | 102.88% | 98.10% |
| | | M3 | 109.79% | 109.07% | 103.09% | 101.32% |
| | 84.5 mg to 104.5 mg/capsule | M_{avg} | 109.3% | 109.0% | 102.9% | 99.1% |
| | | CV | 0.40% | 0.35% | 0.13% | 1.91% |
| | | mg | 103.8 mg/capsule | 103.6 mg/capsule | 97.8 mg/capsule | 94.2 mg/capsule |
| | Sum % EPA and DHA 90% to 110.0% 198 mg to 242 mg/capsule | M1 | 108.74% | 109.01% | 102.70% | 97.58% |
| | | M2 | 108.75% | 108.51% | 102.66% | 97.63% |
| | | M3 | 109.49% | 109.09% | 102.88% | 100.70% |
| | | M_{avg} | 109.0% | 108.9% | 102.8% | 98.6% |
| | | CV | 0.40% | 0.29% | 0.11% | 1.81% |
| | | mg | 240 mg/capsule | 240 mg/capsule | 226 mg/capsule | 217 mg/capsule |
| | Total omega-3 ethyl esters No less than 80% No less than 216 mg/capsule | M1 | 97.80% | 98.21% | 92.22% | 87.80% |
| | | M2 | 97.81% | 97.80% | 92.19% | 87.90% |
| | | M3 | 98.78% | 98.23% | 92.38% | 90.68% |
| | | M_{avg} | 98% | 98% | 92% | 89% |
| | | CV | 0.57% | 0.25% | 0.11% | 1.84% |
| | | mg | 265 mg/capsule | 265 mg/capsule | 249 mg/capsule | 240 mg/capsule |
| Rosuvastatin Dissolution | Rosuvastatin Q = 75.0% in 60 minutes | M1 | 94.25% | 102.25% | 87.16% | 100.63% |
| | | M2 | 93.97% | 91.84% | 88.37% | 90.91% |
| | | M3 | 101.97% | 87.96% | 89.54% | 94.77% |
| | | M4 | 95.90% | 86.61% | 93.87% | 94.16% |
| | | M5 | 89.23% | 94.42% | 82.58% | 102.00% |
| | | M6 | 103.94% | 89.82% | 91.00% | 89.37% |
| | | M_{avg} | 96.5% | 92.2% | 88.8% | 95.3% |
| | | CV | 5.67% | 6.16% | 4.29% | 5.34% |
| Organic impurities in Rosuvastatin | Rosuvastatin ketone: No more than 2.10% | M1 | 0.813% | 1.625% | 1.596% | 1.268% |
| | Rosuvastatin lactone: No more than 6.00% | M1 | 0.32% | 2.475% | 2.66% | 4.00% |
| | Rosuvastatin ethyl ester: No more than 0.50% | M1 | Not detected | Not detected | Not detected | Not detected |
| | Any non-specific degradation product: No more than 0.20% | M1 | 0.018% | 0.042% | 0.069% | 0.123% |
| | Total degradation products: Not more than 3.60% | M1 | 0.042% | 0.022% | 0.125% | 0.370% |
| Microbial limits | Aerobic mesophilic count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence | Not applicable | Absence | Absence |
| Water content | Tablet: No more than 15.00% | X20 | 4.13% | 5.12% | 4.57% | 4.61% |
| | Granules: No more than 15.00% | | 4.61%% | 4.47% | 4.94% | 5.74% |

| | | | | | | |
|---|---|---|---|---|---|---|
| EPA = Eicosapentaenoic acid ethyl ester DHA = Docosahexaenoic acid ethyl ester | | | | | | |

**Table 3. Results of stability study for batch DFF2208-23**

| **Determination** | **Specification** | **Sample No.** | **Initial Mar 3, 2023** | **1 month Apr 3, 2023** | **3 months June 3, 2023** | **6 months September 3, 2023** |
|---|---|---|---|---|---|---|
| Rosuvastatin Content | Rosuvastatin 90.0% to 110.0% 9.0 to 11.0 mg/capsule | M1 | 100.44% | 98.36% | 96.25% | 92.37% |
| | | M2 | 100.32% | 98.84 | 96.64% | 92.52% |
| | | M3 | 101.03% | 98.86 | 96.92% | 92.14% |
| | | M_{avg} | 100.6% | 98.7% | 96.6% | 92.3% |
| | | CV | 0.38% | 0.29% | 0.35% | 0.21% |
| | | mg | 10.1 mg/capsule | 9.9 mg/capsule | 9.7 mg/capsule | 9.2 mg/capsule |
| | EPA 90% to 110.0% 112.5 mg to 137.5 mg per capsule | M1 | 108.34% | 105.18% | 102.21% | 97.23% |
| | | M2 | 108.10% | 106.10% | 102.43% | 99.11% |
| | | M3 | 108.58% | 106.93% | 103.04% | 100.92% |
| | | M_{avg} | 108.3% | 106.1% | 102.6% | 99.1% |
| | | CV | 0.22% | 0.83% | 0.42% | 1.86% |
| | | mg | 135.4 mg/capsule | 132.6 mg/capsule | 128.2 mg/capsule | 123.9 mg/capsule |
| | DHA 90.0% to 110.0% 84.5 mg to 104.5 mg/capsule | M1 | 108.06% | 102.98% | 101.99% | 98.33% |
| | | M2 | 107.52% | 103.97% | 102.40% | 100.15% |
| | | M3 | 108.59% | 104.75% | 103.14% | 101.58% |
| Omega-3 Content | | M_{avg} | 108.1% | 103.9% | 102.5% | 100.0% |
| | | CV | 0.50% | 0.85% | 0.57% | 1.63% |
| | | mg | 102.7 mg/capsule | 98.7 mg/capsule | 97.4 mg/capsule | 95.0 mg/capsule |
| | Sum % EPA and DHA 90% to 110.0% 198 mg to 242 mg/capsule | M1 | 107.93% | 103.95% | 101.84% | 97.58% |
| | | M2 | 107.56% | 104.90% | 102.14% | 97.63% |
| | | M3 | 108.30% | 105.71% | 102.88% | 100.70% |
| | | M_{avg} | 107.9% | 104.9% | 102.8% | 98.6% |
| | | CV | 0.34% | 0.80% | 0.11% | 1.81% |
| | | mg | 237 mg/capsule | 231 mg/capsule | 226 mg/capsule | 217 mg/capsule |
| | Total omega-3 ethyl esters No less than 80% No less than 216 mg/capsule | M1 | 97.07% | 93.67% | 91.48% | 87.71% |
| | | M2 | 96.75% | 94.53% | 91.73% | 89.37% |
| | | M3 | 97.41% | 95.27% | 92.33% | 90.84% |
| | | M_{avg} | 97% | 94% | 92% | 89% |
| | | CV | 0.34% | 0.91% | 0.48% | 1.75% |
| | | mg | 262 mg/capsule | 255 mg/capsule | 248 mg/capsule | 241 mg/capsule |
| Rosuvastatin Dissolution | Rosuvastatin Q = 75.0% in 60 minutes | M1 | 99.36% | 89.80% | 90.89% | 89.22% |
| | | M2 | 90.52% | 96.66% | 93.93% | 89.55% |
| | | M3 | 90.94% | 102.36% | 87.81% | 83.39% |
| | | M4 | 92.09% | 90.19% | 90.99% | 92.61% |
| | | M5 | 84.47% | 90.86% | 93.72% | 87.46% |
| | | M6 | 96.31% | 90.09% | 87.72% | 84.39% |
| | | M_{avg} | 92.3% | 93.3% | 90.8% | 87.8% |
| | | CV | 5.57% | 5.50% | 2.99% | 3.93% |
| Organic impurities in Rosuvastatin | Rosuvastatin ketone: No more than 2.10% | M1 | 0.535% | 1.706% | 1.676% | 1.168% |
| | Rosuvastatin lactone: No more than 6.00% | M1 | 0.49% | 2.73% | 2.59% | 4.62% |
| | Rosuvastatin ethyl ester: No more than 0.50% | M1 | Not detected | Not detected | Not detected | Not detected |
| | Any non-specific degradation product: No more than 0.20% | M1 | 0.011% | 0.045% | 0.068% | 0.156% |
| | Total degradation products: No more than 3.60% | M1 | 0.033% | 0.231% | 0.135% | 0.393% |
| Microbial limits | Aerobic mesophile count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence | Not applicable | Absence | Absence |
| Water content | Tablet: No more than 15.00% | X20 | 3.99% | 5.08% | 4.64% | 4.61% |
| | Granules: No more than 15.00% | | 4.59% | 4.30% | 5.04% | 5.19% |

| | | | | | | |
|---|---|---|---|---|---|---|
| EPA = Eicosapentaenoic acid ethyl ester DHA = Docosahexaenoic acid ethyl ester | | | | | | |

**Table 4. Results of stability study for batch DFF2208-24**

| **Determination** | **Specification** | **Sample No.** | **Initial Mar 3, 2023** | **1 month Apr 3, 2023** | **3 months June 3, 2023** | **6 months September 3, 2023** |
|---|---|---|---|---|---|---|
| Rosuvastatin Content | Rosuvastatin 90.0% to 110.0% 9.0 to 11.0 mg/capsule | M1 | 100.29% | 98.94% | 96.07% | 90.89% |
| | | M2 | 99.87% | 99.26% | 96.39% | 90.87% |
| | | M3 | 100.06% | 98.98% | 96.67% | 90.34% |
| | | M_{avg} | 100.1% | 99.1% | 96.4 | 90.7% |
| | | CV | 0.69% | 0.17% | 0.31% | 0.34% |
| | | mg | 10.0 mg/capsule | 9.9 mg/capsule | 9.6 mg/capsule | 9.1 mg/capsule |
| Omega-3 Content | EPA 90% to 110.0% 112.5 mg to 137.5 mg per capsule | M1 | 108.90% | 106.32% | 102.54% | 99.72% |
| | | M2 | 107.96% | 107.30% | 101.93% | 102.32% |
| | | M3 | 108.09% | 106.86% | 102.29% | 101.29% |
| | | M_{avg} | 108.3% | 106.8% | 102.3% | 101.1% |
| | | CV | 0.47% | 0.46% | 0.30% | 1.30% |
| | | mg | 135.4 mg/capsule | 133.5 mg/capsule | 127.8 mg/capsule | 126.4 mg/capsule |
| | DHA 90.0% to 110.0% 84.5 mg to 104.5 mg/capsule | M1 | 109.99% | 104.33% | 103.03% | 101.09% |
| | | M2 | 107.75% | 105.12% | 102.11% | 104.29% |
| | | M3 | 107.76% | 104.85% | 102.57% | 103.23% |
| | | M_{avg} | 108.5% | 104.8% | 102.6% | 102.9% |
| | | CV | 1.19% | 0.57% | 0.45% | 1.58% |
| | | mg | 102.7 mg/capsule | 99.5 mg/capsule | 97.4 mg/capsule | 97.7 mg/capsule |
| | Sum % EPA and DHA 90% to 110.0% 198 mg to 242 mg/capsule | M1 | 109.07% | 105.18% | 102.48% | 100.04% |
| | | M2 | 107.58% | 106.07% | 101.74% | 102.90% |
| | | M3 | 107.66% | 105.71% | 102.14% | 101.86% |
| | | M_{avg} | 108.1% | 105.7% | 102.1% | 101.6% |
| | | CV | 0.78% | 0.43% | 0.36% | 1.42% |
| | | mg | 238 mg/capsule | 232 mg/capsule | 225 mg/capsule | 224 mg/capsule |
| | Total omega-3 ethyl esters No less than 80% No less than 216 mg/capsule | M1 | 98.36% | 94.78% | 92.01% | 90.05% |
| | | M2 | 96.76% | 95.60% | 91.35% | 92.64% |
| | | M3 | 96.83% | 95.32% | 91.66% | 91.71% |
| | | M_{avg} | 97% | 95% | 92% | 91% |
| | | CV | 0.93% | 0.17% | 0.36% | 1.43% |
| | | mg | 263 mg/capsule | 257 mg/capsule | 248 mg/capsule | 247 mg/capsule |
| Rosuvastatin Dissolution | Rosuvastatin Q = 75.0% in 60 minutes | M1 | 98.93% | 101.17% | 91.41% | 91.23% |
| | | M2 | 83.74% | 90.70% | 93.92% | 91.19% |
| | | M3 | 87.08% | 95.48% | 91.71% | 82.71% |
| | | M4 | 96.30% | 89.70% | 91.64% | 88.94% |
| | | M5 | 102.74% | 96.21% | 94.13% | 90.03% |
| | | M6 | 98.17% | 86.37% | 92.09% | 88.8% |
| | | M_{avg} | 94.5% | 93.3% | 92.5% | 87.8% |
| | | CV | 7.85% | 5.73% | 1.31% | 3.57% |
| Organic impurities in Rosuvastatin | Rosuvastatin ketone: No more than 2.10% | M1 | 0.690% | 1.768% | 1.969% | 1.121% |
| | Rosuvastatin lactone: No more than 6.00% | M1 | 0.41% | 3.39% | 3.29% | 4.66% |
| | Rosuvastatin ethyl ester: No more than 0.50% | M1 | Not detected | Not detected | Not detected | Not detected |
| | Any non-specific degradation product: No more than 0.20% | M1 | 0.015% | 0.046% | 0.067% | 0.156% |
| | Total degradation products: No more than 3.60% | M1 | 0.036% | 0.307% | 0.155% | 0.392% |
| Microbial limits | Aerobic mesophilic count: No more than 10³ CFU/g | X20 | Less than 10 CFU/g | Not applicable | Less than 10 CFU/g | Less than 10 CFU/g |
| | Filamentous fungi and yeasts: No more than 10² CFU/g | | Less than 10 CFU/g | Not applicable | Less than 10 CFU/g | Less than 10 CFU/g |
| | Absence of pathogens: Absence of *Escherichia coli* | | Absence | Not applicable | Absence | Absence |
| Water content | Tablet: No more than 15.00% | X20 | 3.94% | 5.42% | 4.60% | 4.72% |
| | Granules: No more than 15.00% | | 4.13% | 4.33% | 5.00% | 5.19% |

| | | | | | | |
|---|---|---|---|---|---|---|
| EPA = Eicosapentaenoic acid ethyl ester DHA = Docosahexaenoic acid ethyl ester | | | | | | |

From the results set forth and described above, it can be concluded that the tested batches (DFF22208-22, DFF2208-23, and DFF2208-24) demonstrated that they retained their chemical and physical characteristics within the established specifications, thus ensuring their quality, efficacy, and safety.

### Example 3. Comparative bioavailability study

A bioavailability study was conducted to compare the bioavailability of rosuvastatin in two different medicaments that comprise the drug rosuvastatin and rosuvastatin/omega-3, the first component (rosuvastatin) at the same dose.

To this end, a single-dose, two-sequence, two-period, two-treatment, randomized, 2x2 crossover, single-blind study was conducted under fasting conditions in a healthy Mexican population of both genders, with a washout period of 1 week (7 days) between each period to allow for drug elimination.

The reference medicament was as follows:
- Crestor^{®}: Tablet comprising 10 mg rosuvastatin, manufactured by IPR Pharmaceuticals Incorporated for AstraZeneca UK Limited, packaged and distributed by AstraZeneca, S.A. de C.V.

As indicated above, the test medicament corresponds to a pharmaceutical composition according to one embodiment of the present invention, particularly a pharmaceutical composition as described in Example 1 and Table 1.

The study was conducted in accordance with the General Health Law, Regulations of the General Health Law on Health Research, NOM-177-SSA1-2013, NOM-012-SSA3-2012, NOM-220-SSA1-2016 and its amendment, as well as the provisions of CONBIOÉTICA, in accordance with the Declaration of Helsinki, GCP (ICH E6-R2), the International Ethical Guidelines for Health-Related Research Involving Humans (CIOMS), GLP, other applicable legal provisions in Mexico, and protocol ROS/A672-22. The COFEPRIS authorization number is 223301410B0273/2022.

Prior to any process related to the clinical trial, the research subjects signed the Informed Consent Form after having received complete written and verbal information about the aspects involved in the trial, thus confirming their voluntary participation.

To determine the pharmacokinetic curve of the tested substance (rosuvastatin), each subject, after fasting for 10 hours, had a blood sample taken at timepoint 0.00, after a catheter had been placed, they were then administered a 10 mg/300 mg rosuvastatin/omega-3 tablet, or 10 mg rosuvastatin orally, depending on prior randomization (test or reference medicament), with 250 mL of water at room temperature. Subsequently, another 20 samples were taken at the following timepoints: 0.50, 1.00, 1.50, 2.00, 2.50, 3.00, 3.50, 4.00, 4.50, 5.00, 5.50, 3.00, 7.00, 7.00, 8.00, 9.00, 10.00, 12.00, 24.00, 48.00, and 72.00 hours in each period of the trial.

### Population

Based on the literature and intra-subject variability, a minimum sample size of 30 subjects was estimated, so the trial was conducted in one group and two periods, starting with 36 subjects and ending with 33 of the 36 research subjects requested. 21 female subjects and 12 male subjects.

Of the two dropouts from the study, one withdrew because they did not show up for the second period of hospitalization, and the second because they tested positive for pregnancy during the second period of hospitalization.

### Treatment administered

Table 5 shows the characteristics of the treatments administered.

**Table 5. Treatments administered, description of medicaments**

| **Medication details** | **Test medicament** | **Reference medicament** |
|---|---|---|
| Manufacturer | Laboratorios Silanes, S.A. de C.V. | IPR Pharmaceuticals Incorporated |
| Pharmaceutical form | Capsule | Tablet |
| International nonproprietary name | Rosuvastatin/Omega-3 | Rosuvastatin |
| Distinctive name | N/A | Crestor^{®} |
| Expiration date | 08/2024 | 09/2024 |
| Batch number | DFF2208-22 | 177M2003 SSA IV |
| Dose | 10 mg/300 mg | 10 mg |
| Dosage regimen | Single dose | Single dose |

### Vital signs

Vital signs, including blood pressure, respiratory rate, heart rate, and temperature, were taken during hospitalization (approximately at 16:00 h), prior to dose administration (approximately at 07:00 h), post-dosing (at 09:00, 13:00, and 19:00 h), before discharge (approximately at 07:00 h), and during outpatient visits (approximately at 07:00 h).

### Adverse events

From the signing of the informed consent form to the discharge of the research subjects, a total of 10 suspected adverse reactions and/or adverse events occurred, which are described in Table 6.

**Table 6. Description of adverse events**

| Subject code | Treatment administered | Patient gender | Patient age (years) | Adverse event | Severity of report | Severity | Causality | Expected or unexpected event |
|---|---|---|---|---|---|---|---|---|
| B15 | Reference medicament | Female | 33 | Headache | Not serious | Moderate | Possible | Expected |
| B18 | Test medicament | Female | 27 | Headache | Not serious | Moderate | Possible | Expected |
| A05 | Reference medicament | Female | 23 | Headache | Not serious | Moderate | Possible | Expected |
| A07 | Reference medicament | Female | 32 | Hyaline rhinorrhea | Not serious | Mild | Not related | Unexpected |
| A11 | Reference medicament | Female | 22 | Pharyngitis | Not serious | Moderate | Not related | Unexpected |
| A15 | Reference medicament | Female | 28 | Hyaline rhinorrhea | Not serious | Mild | Not related | Unexpected |
| B18 | Test medicament | Female | 27 | Hyaline rhinorrhea | Not serious | Moderate | Not related | Unexpected |
| B11 | Reference medicament | Male | 20 | Pharyngotonsillitis | Not serious | Moderate | Not related | Unexpected |
| A02 | Test medicament | Female | 30 | Myalgia | Not serious | Moderate | Possible | Expected |
| A12 | Reference medicament | Female | 22 | Pregnancy | * | ** | ** | ** |

| | | | | | | | | |
|---|---|---|---|---|---|---|---|---|
| * Exposure to the drug during pregnancy. **Follow-up | | | | | | | | |

It is important to mention that all research subjects attended approximately 01 week after the last sample of the last period for a clinical interview and follow-up on possible adverse events and suspected adverse reactions. All 36 participating research subjects were clinically satisfactory and were discharged as healthy subjects. Subject A12 was discharged from the trial but continues to be monitored due to pregnancy.

### Pharmacokinetic bioavailability between treatments

To compare the bioavailability of rosuvastatin in two different medicaments, their plasma pharmacokinetic patterns, area under the curve from time zero to the last determination (ABC₀₋ₜ ), and maximum plasma concentration (Cₘₐₓ ) were assessed.

The results of the comparative bioavailability are shown in Table 7.

**Table 7. Pharmacokinetic patterns for the reference medicament (R) and the test medicament (P) in 33 research subjects**

| Pharmacokinetic parameter | Arithmetic Mean | | Standard Deviation | | Coefficient of Variation | | Geometric Mean | | Minimum | | Median | | maximum | |
|---|---|---|---|---|---|---|---|---|---|---|---|---|---|---|
| | R | P | R | P | R | P | R | P | R | P | R | P | R | P |
| ABC₀₋ₜ (ng/mL*h) | 92.463 | 84.557 | 53.222 | 45.530 | 0.575 | 0.538 | 79.471 | 73.978 | 31.380 | 31.926 | 85.964 | 71.581 | 286.328 | 204.839 |
| ABC_{0-∞} (ng/mL*h) | 105.437 | 98.493 | 56.186 | 48.194 | 0.532 | 0.489 | 92.597 | 88.308 | 37.446 | 39.863 | 98.833 | 89.768 | 302.223 | 215.500 |
| Cmax (ng/mL) | 10.537 | 10.080 | 4.813 | 5.824 | 0.456 | 0.577 | 9.400 | 8.567 | 3.297 | 3.018 | 10.614 | 7.729 | 20.426 | 22.175 |
| Tmax (h) | 4.166 | 4.136 | 1.108 | 0.721 | 0.266 | 0.174 | 4.011 | 4.059 | 2.000 | 2.000 | 4.500 | 4.500 | 8.000 | 5.000 |
| Kₑ (1/h) | 0.081 | 0.081 | 0.046 | 0.048 | 0.566 | 0.596 | 0.070 | 0.069 | 0.021 | 0.019 | 0.079 | 0.078 | 0.241 | 0.210 |
| T_{1/2} (h) | 11.431 | 11.773 | 6.763 | 7.433 | 0.591 | 0.631 | 9.796 | 9.968 | 2.873 | 3.294 | 8.711 | 8.839 | 32.690 | 36.385 |

Table 8 provides the results of log-transformation (log-base e) for the pharmacokinetic parameters.

**Table 8. Log-transformation of estimated pharmacokinetic parameters.**

| Pharmacokinetic parameter | Arithmetic Mean | | Standard Deviation | | Coefficient of Variation | | Geometric Mean | | Minimum | | Median | | maximum | |
|---|---|---|---|---|---|---|---|---|---|---|---|---|---|---|
| | R | P | R | P | R | P | R | P | R | P | R | P | R | P |
| ABC₀₋ₜ | 4.375 | 4.303 | 0.565 | 0.524 | 0.129 | 0.121 | 4.339 | 4.272 | 3.446 | 3.463 | 4.453 | 4.270 | 5.657 | 5.322 |
| ABC_{0-∞} | 4.528 | 4.480 | 0.520 | 0.471 | 0.115 | 0.105 | 4.498 | 4.456 | 3.622 | 3.685 | 4.593 | 4.497 | 5.711 | 5.372 |
| Cmax | 2.240 | 2.148 | 0.504 | 0.585 | 0.225 | 0.272 | 2.180 | 2.066 | 1.193 | 1.104 | 2.362 | 2.045 | 3.016 | 3.099 |

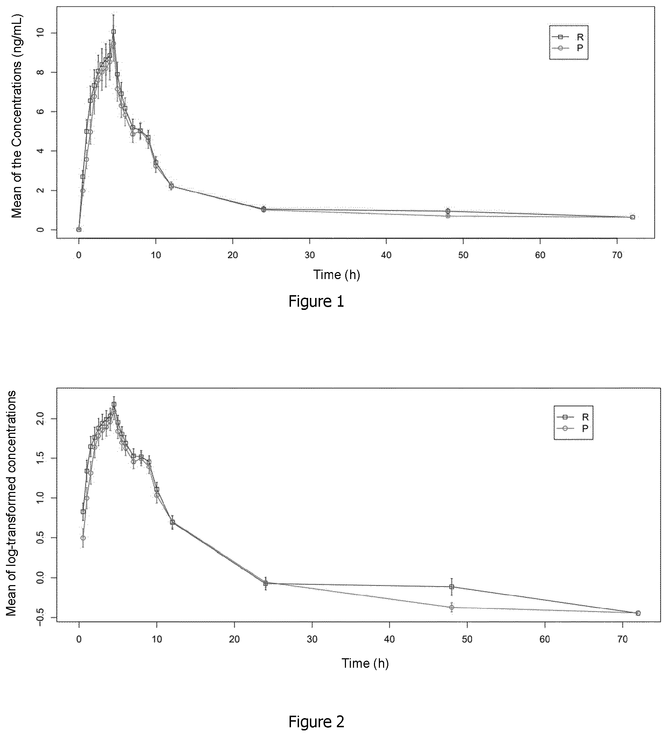

On the other hand, Figure 1 is a plot of the mean plasma concentration versus sampling time, while Figure 2 depicts a plot of the mean of log-transformed rosuvastatin concentrations (ng/mL) in subjects who received the reference medicament (R) and the test medicament (P). It can be seen that the trend for the test medicament is very similar to that of the reference medicament; in fact, it appears that the plasma concentration of rosuvastatin when administered in the test medicament is slightly higher than in the reference medicament.

Finally, inferential statistics were performed to study the possible equivalence of the bioavailability of the active ingredient rosuvastatin. The confidence intervals for the log-transformation of parameters ABC₀₋ₜ, ABC_{0-∞}, and Cₘₐₓ are detailed in Table 9.

**Table 9. Confidence intervals of the study**

| **Parameter*** | **Classic interval** | | **Power** |
|---|---|---|---|
| ABC₀₋ₜ | 84.7801 | 102.0757 | 0.9881 |
| ABC_{0-∞} | 87.5901 | 103.7286 | 0.9954 |
| Cₘₐₓ | 82.0720 | 100.4918 | 0.9751 |

| | | | |
|---|---|---|---|
| *Log-base: e | | | |

According to the bioavailability equivalence tests used and suggested by standard NOM-177-SSA1-2013, in the active ingredient rosuvastatin, the 90% confidence intervals (IC90) estimated from the log-transformation of pharmacokinetic parameters: ABC₀₋ₜ, ABC_{0-∞}, and Cₘₐₓ were found to be in the range of 80-125%, as cited in the protocol. As additional information, the IC90 estimated from the log-transformation of ABC_{0-∞} was also found to be in the range of 80-125%.

Based on the above, the test medicament, i.e., the pharmaceutical composition of the present invention, which in one embodiment comprises rosuvastatin/omega-3 at a dose of 10 mg/300 mg, is equivalent in bioavailability to the reference medicament Crestor^{®}, which is a tablet comprising 10 mg rosuvastatin.

According to the matter described above, it can be seen that the pharmaceutical composition comprising rosuvastatin and omega-3 has been conceived to provide a stable, immediate-release pharmaceutical form, and it will be evident to any person skilled in the art that embodiments with regard to a pharmaceutical composition comprising rosuvastatin and omega-3, process for preparing the same, and uses thereof as described above and illustrated in the accompanying drawings are only illustrative and not limiting of the present invention, since numerous changes of consideration are possible in their details without departing from the scope of the invention.

Therefore, the present invention shall not be considered as restricted except for what is required by the prior art and the scope of the attached claims.

## Claims

1. A pharmaceutical composition **characterized by** comprising: (a) a first phase comprising rosuvastatin, and (b) a second phase comprising omega-3,
wherein the first phase comprising rosuvastatin is in the form of a coated tablet and the second phase comprising omega-3 is in the form of granules.

2. The pharmaceutical composition according to claim 1, further comprising pharmaceutically acceptable excipients selected from the group consisting of diluents, stabilizing agents, disintegrants, glidants, lubricants, solvents, coatings, adsorbents, antioxidants, and surfactants.

3. The pharmaceutical composition according to claim 1, wherein the first phase comprising rosuvastatin further comprises at least one of a diluent selected from the group consisting of lactose monohydrate, microcrystalline cellulose, anhydrous calcium phosphate, and corn starches; a stabilizing agent selected from the group consisting of phosphate derivatives including anhydrous dibasic calcium phosphate, and aluminum magnesium silicate; a disintegrant selected from the group consisting of croscarmellose sodium, cellulose derivatives including hydroxypropyl celluloses, carboxymethyl celluloses, microcrystalline cellulose, povidone derivatives including plasdones, starch derivatives including pregelatinized starch, sodium starch glycolate, and corn starch; a glidant selected from the group consisting of colloidal silicon dioxide, cellulose derivatives including carboxymethyl celluloses, microcrystalline cellulose, starch derivatives including pregelatinized starch and corn starch, silicate derivatives including magnesium silicate, magnesium trisilicate, and talc; a lubricant selected from the group consisting of stearate derivatives including zinc stearate, magnesium stearate, calcium stearate, stearic acid, monostearates, stearyl fumarate, sulfated derivatives including magnesium lauryl sulfate, and talc; and a solvent which is water.

4. The pharmaceutical composition according to claim 3, wherein the first phase comprising rosuvastatin comprises two lubricants.

5. The pharmaceutical composition according to claim 3 or 4, wherein the first phase comprising rosuvastatin further comprises lactose monohydrate as a diluent, dibasic calcium phosphate as a stabilizing agent, croscarmellose sodium as a disintegrant, colloidal silicon dioxide as a glidant, talc as a first lubricant, and zinc stearate as a second lubricant.

6. The pharmaceutical composition according to any of claims 3 to 5, wherein the first phase comprising rosuvastatin further comprises:
- rosuvastatin or a pharmaceutically acceptable salt thereof as an active ingredient in a dose of from 10 mg to 20 mg;
- lactose monohydrate in an amount of from 40% to 80% by weight of the tablet;
- dibasic calcium phosphate in an amount of from 0.26% to 2% by weight of the tablet;
- croscarmellose sodium in an amount of from 0.5% to 5% by weight of the tablet;
- colloidal silicon dioxide in an amount of from 0.5% to 6% by weight of the tablet;
- talc in an amount of from 0.2% to 5% by weight of the tablet; and
- zinc stearate in an amount of from 0.5% to 5% by weight of the tablet.

7. The pharmaceutical composition according to any of claims 3 to 6, wherein the coating of the first phase comprising rosuvastatin is polyvinyl acetate and is present in an amount of from 0.5% to 10% by weight of the tablet.

8. The pharmaceutical composition according to claim 7, wherein the coating is present in an amount of from 2% to 6% by weight of the composition.

9. The pharmaceutical composition according to claim 1, wherein the second phase comprising omega-3 further comprises at least one of an adsorbent selected from the group consisting of phosphate derivatives including dibasic calcium phosphate, aluminum magnesium silicate, aluminum magnesium metasilicate, and cellulose derivatives including microcrystalline cellulose; a diluent selected from the group consisting of cellulose derivatives including microcrystalline cellulose, silicified microcrystalline cellulose, phosphate derivatives including dibasic calcium phosphate, and starch derivatives including pregelatinized starch and corn starch; an antioxidant selected from the group consisting of propyl gallate, tert-butylhydroquinone (TBHQ), butylhydroxytoluene (BHT), butylhydroxyanisole (BHA), and alpha-tocopherols; a disintegrant selected from the group consisting of cellulose derivatives including hydroxypropyl celluloses, carboxymethyl celluloses, microcrystalline cellulose, povidone derivatives including crospovidone and plasdone, and starch derivatives including pregelatinized starch, sodium starch glycolate, and corn starch; and a surfactant selected from the group consisting of sodium lauryl sulfate, poloxamers, polyoxyethylene castor oil derivatives, benzalkonium chloride, benzetonium chloride, polyoxyethylene alkyl ethers, and polyoxyethylene sorbitan fatty acid esters.

10. The pharmaceutical composition according to claim 9, wherein the second phase comprising omega-3 further comprises aluminum magnesium metasilicate as an adsorbent, silicified microcrystalline cellulose as a diluent, propyl gallate as an antioxidant, crospovidone as a disintegrant, and sodium lauryl sulfate as a surfactant.

11. The pharmaceutical composition according to claim 10, wherein the second phase comprising omega-3 further comprises:
- omega-3 in an amount of 300 mg ± 0.5 mg;
- aluminum magnesium metasilicate in an amount of from 20% to 50% by weight of the granules;
- silicified microcrystalline cellulose in an amount of from 1% to 5% by weight of the granules;
- propyl gallate in an amount of from 0.5% to 5% by weight of the granules;
- crospovidone in an amount of from 3% to 10% by weight of the granules; and
- sodium lauryl sulfate in an amount of from 0.5% to 5% by weight of the granules.

12. The pharmaceutical composition according to claim 11, wherein 300 mg of omega-3 is equivalent to 220 mg of fatty acid esters.

13. The pharmaceutical composition according to claim 11, wherein 300 mg of omega-3 corresponds to 125 mg of eicosapentaenoic acid (EPA) ethyl ester and to 95 mg of docosahexaenoic acid (DHA) ethyl ester.

14. The pharmaceutical composition according to any of claims 1 to 13, wherein the pharmaceutical composition is a solid form.

15. The pharmaceutical composition according to claim 14, wherein the solid pharmaceutical form is a capsule.

16. The pharmaceutical composition according to any of claims 1 to 14, wherein it comprises:
(a) a first phase comprising:
- 10.4 mg rosuvastatin calcium;
- 50.20 mg lactose monohydrate;
- 1.20 mg anhydrous dibasic calcium phosphate;
- 3.5 mg croscarmellose sodium;
- 0.40 mg colloidal silicon dioxide;
- 3.6 mg talc; and
- 0.70 mg zinc stearate;
wherein the first phase of rosuvastatin is in the form of a coated tablet;
(b) a second phase comprising:
- 300 mg omega-3;
- 171.50 mg aluminum magnesium metasilicate;
- 21 mg siliconized microcrystalline cellulose;
- 15 mg propyl gallate;
- 49.5 mg crospovidone; and
- 15 mg sodium lauryl sulfate;
where the second phase of omega-3 is in the form of granules; and
the pharmaceutical form containing the first phase of rosuvastatin and the second phase of omega-3 is a capsule.

17. A process for manufacturing a pharmaceutical composition as described in any of claims 1 to 16, **characterized in that** it comprises the following stages:
a) providing a first phase comprising rosuvastatin, wherein the first phase comprising rosuvastatin is in the form of a coated tablet;
b) providing a second phase comprising omega-3, wherein the second phase comprising omega-3 is in the form of granules; and
c) encapsulating the first phase comprising rosuvastatin and the second phase comprising omega-3.

18. The process according to claim 17, wherein the first phase comprising rosuvastatin is prepared by the following steps:
- mixing rosuvastatin with a lubricant, a disintegrant, a stabilizing agent, and a diluent;
- lubricating the above mixture with at least one lubricant;
- compressing the lubricated mixture to obtain a rosuvastatin tablet;
- coating the tablet.

19. The process according to claim 18, wherein the diluent is selected from the group consisting of lactose monohydrate, microcrystalline cellulose, anhydrous calcium phosphate, and corn starches; the stabilizing agent is selected from the group consisting of phosphate derivatives including anhydrous dibasic calcium phosphate, and aluminum magnesium silicate; the disintegrant is selected from the group consisting of croscarmellose sodium, cellulose derivatives including hydroxypropyl celluloses, carboxymethyl celluloses, microcrystalline cellulose, povidone derivatives including plasdones, starch derivatives including pregelatinized starch, sodium starch glycolate, and corn starch; the glidant is selected from the group consisting of colloidal silicon dioxide, cellulose derivatives including carboxymethyl celluloses, microcrystalline cellulose, starch derivatives including pregelatinized starch and corn starch, silicate derivatives including magnesium silicate, magnesium trisilicate, and talc; the lubricant is selected from the group consisting of stearate derivatives including zinc stearate, magnesium stearate, calcium stearate, stearic acid, monostearates, stearyl fumarate, sulfated derivatives including magnesium lauryl sulfate, and talc; the solvent is water; and the coating is selected from the group consisting of cellulose derivatives including hydroxypropyl methylcellulose, hydroxypropyl cellulose, carboxymethyl celluloses, polyvinyl derivatives including polyvinyl alcohol, polyethylene glycol, povidones in all K grades and derivatives thereof, and polyvinyl acetate coatings.

20. The process according to claim 17, wherein the second phase comprising omega-3 is prepared by the following steps:
- mixing the omega-3 with an adsorbent, an antioxidant, a surfactant, a diluent, and a disintegrant; and
- granulating.

21. The process according to claim 20, wherein the adsorbent is selected from the group consisting of phosphate derivatives including dibasic calcium phosphate, aluminum magnesium silicate, aluminum magnesium metasilicate, and cellulose derivatives including microcrystalline cellulose; the diluent is selected from the group consisting of cellulose derivatives including microcrystalline cellulose, silicified microcrystalline cellulose, phosphate derivatives including dibasic calcium phosphate, and starch derivatives including pregelatinized starch and corn starch; the antioxidant is selected from the group consisting of propyl gallate, tert-butylhydroquinone (TBHQ), butylhydroxytoluene (BHT), butylhydroxyanisole (BHA), and alpha-tocopherols; the disintegrant is selected from the group consisting of cellulose derivatives including hydroxypropyl celluloses, carboxymethyl celluloses, microcrystalline cellulose, povidone derivatives including crospovidone and plasdone, and starch derivatives including pregelatinized starch, sodium starch glycolate, and corn starch; and the surfactant is selected from the group consisting of sodium lauryl sulfate, poloxamers, polyoxyethylene castor oil derivatives, benzalkonium chloride, benzetonium chloride, polyoxyethylene alkyl ethers, and polyoxyethylene sorbitan fatty acid esters.

22. The process according to any of claims 17 to 21, wherein the encapsulation stage c) comprises putting the first phase comprising rosuvastatin and the second phase comprising omega-3 into a gelatin capsule.

23. A composition as claimed in any of claims 1 to 16 for use in the prevention and treatment of a disorder associated with lipid metabolism selected from the group consisting of dyslipidemia, atherosclerosis, coronary heart disease, cardiovascular disease, and pancreatitis.

24. The composition for use according to claim 23, wherein dyslipidemia is selected from the group consisting of familial hypercholesterolemia, hypertriglyceridemia, familial combined dyslipidemia, familial chylomicronemia syndrome, and hypoalphalipoproteinemia.
